Europäisches Patentamt

European Patent Office

Office européen des brevets

(19)

(11) Veröffentlichungsnummer: **0 167 171 B1**

# EUROPÄISCHE PATENTSCHRIFT

(45) Veröffentlichungstag der Patentschrift: **04.12.91**

(51) Int. Cl.5: **G01N 33/53**, G01N 21/07

(21) Anmeldenummer: **85108355.0**

(22) Anmeldetag: **05.07.85**

(54) **Verfahren und Vorrichtung zur Durchführung analytischer Bestimmungen.**

(30) Priorität: **06.07.84 DE 3425008**

(43) Veröffentlichungstag der Anmeldung:
**08.01.86 Patentblatt 86/02**

(45) Bekanntmachung des Hinweises auf die
Patenterteilung:
**04.12.91 Patentblatt 91/49**

(84) Benannte Vertragsstaaten:
**AT BE CH DE FR GB IT LI LU NL SE**

(56) Entgegenhaltungen:
**EP-A- 0 027 986        EP-A- 0 052 769
EP-A- 0 073 512        EP-A- 0 073 513
US-A- 3 899 296        US-A- 4 279 862**

(73) Patentinhaber: **BOEHRINGER MANNHEIM
GMBH
Patentabteilung, Abt. E Sandhofer Strasse
112-132 Postfach 31 01 20
W-6800 Mannheim 31 Waldhof(DE)**

(72) Erfinder: **Klose, Sigmar Dr.
Breitenloh 7
W-8131 Berg 2(DE)**
Erfinder: **Pasch, Manfred
Anton Bartl-Str. 7
W-8132 Tutzing(DE)**

Erfinder: **Schlumberger, Helmut
Kaiser Heinrich-Str. 23
W-8121 Polling(DE)**
Erfinder: **Kleemann, Wolfgang, Dr.
Alpspitzstr. 7
W-8132 Tutzing(DE)**
Erfinder: **Vieth, Friedhelm
Hauptstr. 11
W-8121 Haunshofen(DE)**

(74) Vertreter: **Weickmann, Heinrich, Dipl.-Ing. et
al
Patentanwälte Dipl.-Ing. H.Weickmann
Dipl.-Phys.Dr. K.Fincke Dipl.-Ing.
F.A.Weickmann Dipl.-Chem. B. Huber Dr.-Ing.
H. Liska Dipl.-Phys.Dr. J. Prechtel Postfach
860820
W-8000 München 86(DE)**

## Beschreibung

Die Erfindung betrifft ein Verfahren zur Durchführung analytischer Bestimmungen unter der Einwirkung einer Zentrifugalkraft und eine zu ihrer Durchführung geeignete Vorrichtung.

Bekannt ist ein Verfahren zur Durchführung analytischer Bestimmungen, bei dem eine Probelösung von einer Aufgabestelle zu einem löslichen Trockenreagenz transportiert wird unter wenigstens teilweiser Auflösung des letzteren, dann zu einer Meßstelle weitertransportiert wird, wobei der Transport durch zwei verschiedene Kräfte derart erfolgt, daß er auf wenigstens einem Teil der Strecke durch eine auf die Lösung wirkende Grenzflächenkraft als erste Kraft bewirkt wird, der zur Regelung der Transportgeschwindigkeit oder der Transportrichtung als zweite Kraft eine Zentrifugalkraft oder/und Druckkraft überlagert wird die, je nachdem, welcher Transportzustand der Flüssigkeit eingestellt werden soll, größer oder kleiner als die erste Kraft gemacht wird (DE-OS 31 34 611). Für ein derartiges Verfahren eignet sich in besonderem Maße die Durchführung auf einem Zentrifugalanalysatorgerät, bei dem der Rotor ein oder mehrere Einsatzelemente aufweist, in denen das lösliche Trockenreagenz eingeschlossen ist, so daß lediglich noch die Probelösung zudosiert werden muß. Aufgrund der Einfachheit und geringen Fehlermöglichkeit bei einem derartigen Verfahren besteht ein Bedarf, möglichst viele verschiedene analytische Bestimmungen durchführen zu können, insbesondere im Rahmen der Bestimmung klinischer Parameter bei der Untersuchung von Körperflüssigkeiten. In neuerer Zeit wurden zahlreiche komplizierte Analysenmethoden entwickelt, beispielsweise auf immunologischer Basis, bei welchen heterogene Phasen auftreten. So basieren beispielsweise viele Methoden des Radioimmunassay (RIA) oder Enzymimmunassay (EIA) auf dem Vorhandensein eines Reaktionspartners in unlöslicher Form, der mit einer löslichen Phase in chemische Wechselwirkung tritt. Ein typisches Beispiel hierfür sind die sogenannten ELISA-Verfahren, bei welchen in heterogener Phase gearbeitet wird und einer der Partner der Immunreaktion an die feste Phase gebunden ist, während der oder die anderen Partner der Immunreaktion in gelöster Phase vorliegen. Derartige Verfahren lassen sich jedoch nicht oder nur schwierig so gestalten, daß sie ohne irgendwelche Manipulationen auf Analysenautomaten, insbesondere auf Zentrifugalanalysatoren, durchgeführt werden können, da in aller Regel mehrere verschiedene Flüssigkeiten benötigt werden bzw. Waschflüssigkeiten und dergleichen gehandhabt werden müssen.

Der Erfindung liegt nun die Aufgabe zugrunde, ein Verfahren der eingangs genannten Art so weiterzuentwickeln, daß es sich auf Methoden anwenden läßt, welche zeitlich in mehreren Stufen ablaufen und auch zur Durchführung derartiger komplizierterer Analysenmethoden, insbesondere von Analysenmethoden, die heterogene Phasen erfordern wie z. B. immunologische Bestimmungen, geeignet ist.

Gelöst wird diese Aufgabe erfindungsgemäß durch ein Verfahren zur Durchführung analytischer Bestimmungen durch Mischen und Inkubieren einer Probelösung mit wenigstens einem Reagenz und Messung eines Parameters im Reaktionsgemisch, wobei man die Probelösung zuerst zu einem löslichen Trockenreagenz transportiert unter wenigstens teilweiser Auflösung des letzteren und dann unter dem Einfluß eines Kraftänderungszyklus weitertransportiert, bei welchem man eine Zentrifugalkraft und eine weitere Kraft derart auf die Lösung einwirken läßt, daß abwechselnd eine Kraft die andere Kraft überwiegt und den Weitertransport und die Transportrichtung bestimmt, welches dadurch gekennzeichnet ist, daß man wenigstens eine weitere Flüssigkeit auf einem Teil des Weges getrennt von der Probelösung und/oder weiteren Flüssigkeiten gleichzeitig transportiert und die Transportwege derart auf die Kraftänderungszyklen abstellt, daß die verschiedenen Flüssigkeiten den gemeinsamen Teil des Transportweges zeitlich getrennt passieren.

Erfindungsgemäß gelingt es, ohne weitere Eingriffe von außen nach Ingangsetzen des Verfahrens, alleine durch die Kraftänderungszyklen, mehrere zeitlich getrennte Vorgänge an einer Reaktionsstelle ablaufen zu lassen.

Die Erfindung geht aus von der Erkenntnis, daß man Zentrifugalkraft und eine weitere Kraft, insbesondere eine Kapillarkraft gleichzeitig auf mehrere Flüssigkeitstransportwege einwirken lassen kann und dabei die Transportwege derart auf die Kraftänderungszyklen abstellen kann, daß ein gemeinsamer Teil des Transportweges von verschiedenen Flüssigkeiten zeitlich getrennt durchlaufen wird.

In einer bevorzugten Ausführungsform des erfindungsgemäßen Verfahrens wird wenigstens einer der getrennten Transportwege so gestaltet, daß die darin transportierte Flüssigkeit bei jedem Kraftänderungszyklus nur einen Teil des vom Weg der anderen Flüssigkeit getrennten Weges zurücklegt. Falls beide Flüssigkeitstransportwege so gestaltet sind, benötigt einer der getrennten Flüssigkeitstransportwege mehr Kraftänderungszyklen bis zum Erreichen des für mindestens zwei der Flüssigkeiten gemeinsamen Transportweges als der andere Transportweg.

Ein Kraftänderungszyklus beim erfindungsgemäßen Verfahren besteht aus einer ersten Kraft in Form einer Zentrifugalkraft und einer zweiten Kraft, vorzugsweise in Form einer Kapillarkraft, von denen abwechselnd die eine die andere überwiegt. Anstelle einer Kapillarkraft kann dabei im Rahmen der Erfindung auch eine geeignete andere Kraft eingesetzt werden, beispielsweise eine Druckkraft, ein elektrisches Feld oder die Schwerkraft. In der Regel läßt sich dabei ein Kraftänderungszyklus schon durch Änderung einer dieser Kräfte, also abwechselnde Erhöhung oder Erniedrigung dieser Kraft derart, daß sie die zweite Kraft übersteigt oder von ihr überwunden wird, ausführen. Beispielsweise genügt es, die Zentrifugalkraft abwechselnd zu erhöhen und zu senken, indem man die Drehzahl des Zentrifugenrotors erhöht bzw. erniedrigt. Jedoch ist es auch möglich, die zweite Kraft entsprechend zu variieren oder Zentrifugalkraft und zweite Kraft so zu ändern, daß sie abwechselnd überwiegen. Als zweite Kraft werden dabei im Rahmen der Erfindung auch mehr als eine mit der Zentrifugalkraft konkurrierende Kraft angesehen. Beispielsweise kann neben der Zentrifugalkraft als erster Kraft auch eine Kapillarkraft und eine Schwerkraft oder/und ein elektrisches Feld die zweite Kraft darstellen.

Die Transportwege für die Probeflüssigkeit und die weitere, im Rahmen der Erfindung verwendete Flüssigkeit sind nun im Rahmen der Erfindung so gestaltet, daß bei jedem Kraftänderungszyklus eine bestimmte Wegstrecke durch die Flüssigkeit innerhalb der Transportwege zurückgelegt wird. Beispielsweise kann die eine Flüssigkeit mit einem oder zwei Kraftänderungszyklen bis zu einem gemeinsamen Teil des Transportweges aller Flüssigkeiten transportiert werden, während die weitere, also die zweite oder dritte usw. Flüssigkeit, drei oder mehr Kraftänderungszyklen benötigt um zur selben gemeinsamen Stelle des Transportweges zu gelangen. Am gemeinsamen Teil des Transportweges ist dann in der Regel der in fester Phase vorliegende Reaktant angeordnet. Bei letzterem handelt es sich beispielsweise um eine Komponente einer Immunreaktion, also ein Antigen, ein Hapten, ein Antikörper, Antikörperfragment oder dergleichen.

In einer besonders bevorzugten Ausführungsform der Erfindung sieht man daher in dem für die verschiedenen Flüssigkeiten gemeinsamen Teil des Transportweges wenigstens ein festes reaktives unlösliches Material vor, beispielsweise also ein Immunreagenz, an welchem man wenigstens zwei Reaktionen zeitlich voneinander getrennt ablaufen läßt, indem man es zuerst mit derjenigen Lösung kontaktiert, die weniger Kraftänderungszyklen bis zum Erreichen dieses unlöslichen reaktiven Materials benötigt, und erst danach die weitere Flüssigkeit bzw. die weiteren Flüssigkeiten damit in Kontakt bringt.

Wird beispielsweise das erfindungsgemäße Verfahren angewendet, um eine in der Probeflüssigkeit vorhandene, als Antigen oder Hapten wirksame Substanz nach dem ELISA-Prinzip zu bestimmen, so kann man die Probelösung zuerst zur Auflösung des Trockenreagenz, welches eine bekannte Menge der zu bestimmenden Substanz in enzymmarkierter Form enthält, transportieren, die erhaltene Mischung, welche eine unbekannte Menge nichtmarkierter Substanz und eine bekannte Menge markierter Substanz enthält, dann zum gemeinsamen Teil des Transportweges weiterleiten, wo in unlöslicher Phase der immunologische Partner der zu bestimmenden Substanz vorliegt. Dort erfolgt eine konkurrierende Bindung von markierter und nichtmarkierter Substanz an die feste Phase in Abhängigkeit vom Mengenverhältnis der beiden zueinander. In einem anschließenden Kraftänderungszyklus wird dann die Probeflüssigkeit weitertransportiert und beispielsweise einer Abfallkammer zugeführt. Währenddessen gelangt die weitere Flüssigkeit, beispielsweise eine Reagenz- und Waschflüssigkeit, nach Durchlaufen einer größeren Anzahl von Kraftänderungszyklen als die Probelösung und zwar vorzugsweise unter Aufteilung in Einzelportionen, ebenfalls zum gemeinsamen Teil des Transportweges, wäscht dort die unlösliche Phase, wozu eine oder mehrere Einzelportionen verwendet werden können, reagiert dabei mit dem Markierungsenzym, welches an die unlösliche Phase gebunden ist unter Farbbildung und wird schließlich ganz oder teilweise zur Meßstelle weitertransportiert und dort ausgemessen, während die nur zum Waschen verwendeten Portionen vorher der Abfallkammer zugeführt und damit dem weiteren Reaktionsablauf entzogen werden.

Wahlweise kann anstelle einer Portionierung der zweiten Flüssigkeit auch eine dritte Flüssigkeit durch einen weiteren Transportweg nach demselben Prinzip zum gemeinsamen Teil des Transportweges transportiert werden, dort eine Farbreaktion bewirken und schließlich zur Meßstelle transportiert werden. In diesem Fall kann die zweite Flüssigkeit nur als Waschflüssigkeit verwendet werden und die dritte Flüssigkeit nur als eine Reagenzflüssigkeit, die eine quantitative Bestimmung des Markierungsenzyms ermöglicht, welches als Bestandteil des von der Probelösung aufgelösten Trockenreagenz zur Markierung der bekannten Menge an zu bestimmender Substanz verwendet wird. Handelt es sich also beispielsweise beim Markierungsenzym um das beim ELISA-Verfahren häufig verwendete Enzym Peroxidase, so besteht die dritte Flüssigkeit zweckmäßig aus einem Peroxidasebestimmungsreagenz, welches in Gegenwart dieses Enzyms einen Farbstoff entwickelt, dessen Menge proportional der Menge an gebunden vorliegendem Enzym und damit auch umgekehrt proportional der Menge an gesuchter Substanz ist, und der an der Meßstelle, z. B. Küvette, ausgemessen wird.

In analoger Weise lassen sich auch die anderen Ausführungsformen des ELISA-Prinzips mit dem erfindungsgemäßen Verfahren durchführen.

In einer anderen Ausführungsform des erfindungsgemäßen Verfahrens wird z. B. zur Bestimmung eines Haptens oder Antigens in der Probeflüssigkeit im löslichen Trockenreagenz ein Antikörper-Enzymkonjugat vorgesehen, welches nach Auflösen durch die Probelösung zur Bildung eines gelösten Komplexes aus Hapten bzw. Antigen und Konjugat und überschüssigem freiem Konjugat führt. Unter dem Einfluß der Kraftänderungszyklen wird diese Mischung dann dem gemeinsamen Teil des Transportweges zugeführt, in welchem weiteres Hapten bzw. Antigen in unlöslicher Phase vorliegt. Noch vorhandenes überschüssiges Konjugat wird dort festgehalten, während die den Hapten-Konjugat-Komplex bzw. Antigen-Konjugat-Komplex enthaltende Flüssigkeit weitertransportiert wird zu einem Trockenreagenz zur Bestimmung des im Konjugat gebundenen Enzyms, dieses auflöst und in einem weiteren Kraftänderungszyklus schließlich zur Meßküvette transportiert wird, in welcher das Enzymreagenz gemessen wird. Als zweite Flüssigkeit kann in diesem Falle eine Waschflüssigkeit verwendet werden, die entweder direkt in die Kammer eingegeben wird, welche das in unlöslicher Phase gebundene Hapten bzw. Antigen enthält oder in eine gesonderte, der Festphasenkammer vorgeschaltete Kammer eingebracht wird. Hierdurch wird eine Vorwaschung der unlöslichen Phase erreicht, welche bei der Lagerung freigesetztes Hapten bzw. Antigen auswäscht, ehe die eigentliche Probeflüssigkeit damit in Berührung kommt. Bei dieser Ausführungsform kann daher der gemeinsame Teil der Flüssigkeitswege gleich dem Gesamtweg sein, den die zweite Flüssigkeit zurücklegt.

Das erfindungsgemäße Verfahren läßt zahlreiche Varianten zu, die aus der folgenden Beschreibung in Verbindung mit der Zeichnung erkennbar sind.

Aus EP-A-0 073 513 ist ein Rotoreinsatzelement bekannt, das eine Probenauftragskammer, verschiedene Reagenzfelder, wenigstens eine Mischventilkammer und eine Meßkammer, die zusammen einen Probenflüssigkeitstransportweg bilden, der von radial innen nach radial weiter außen führt wenn das Einsatzelement auf dem Rotor befestigt ist. Ein derartiges Rotoreinsatzelement eignet sich nicht für die Durchführung des erfindungsgemäßen Verfahrens, da es keine Möglichkeit bietet, wenigstens eine weitere Flüssigkeit auf einem Teil des Weges getrennt von der Probelösung zu transportieren.

In der Zeichnung wird ein zur einmaligen Verwendung bestimmtes Einsatzelement (Disposable) für einen Zentrifugalanalysator schematisch in vergrößerter Form dargestellt, welches zur Ausführung des Verfahrens der Erfindung geeignet ist. In der Zeichnung stellen dar:

Fig. 1    eine schematische Zeichnung einer ersten Ausführungsform,

Fig. 2    eine schematische Zeichnung einer anderen Ausführungsform des Einsatzelementes.

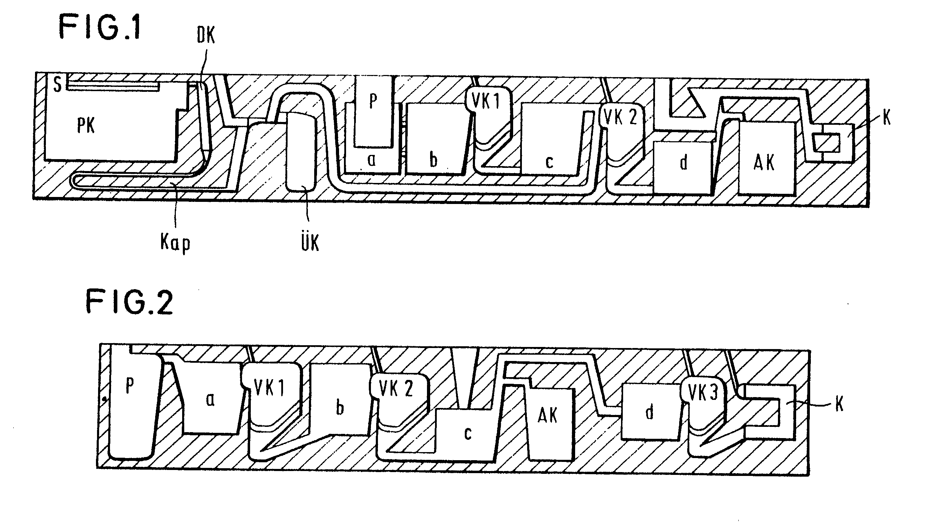

Fig. 1 zeigt ein derartiges Einsatzelement mit zwei voneinander getrennten Flüssigkeitswegen. Der erste Flüssigkeitsweg besteht aus einer Probekammer (P), die mit einer Kammer (a) in Verbindung steht, welche gegebenenfalls mit einem saugfähigen Material, z. B. einem Vlies gefüllt ist. Kammer (a) steht mit Kammer (b), die ebenfalls mit einem Vliesmaterial gefüllt ist, in Verbindung. Kammer (b) kann weggelassen werden. Sie wird normalerweise nicht benötigt, erweitert aber den Einsatzbereich insbesondere bei kompliziert zusammengesetzten Reagenzien, die miteinander unerwünscht zur Reaktion neigende Bestandteile enthalten. Der weitere Flüssigkeitsweg geht zur Ventilkammer (VK1) und von dort über die Kammer (c), die wiederum mit einem saugfähigen Material gefüllt ist, zum Beginn des gemeinsamen Teils der Flüssigkeitswege, der Ventilkammer (VK2). Für einfache Reaktionen sind Kammer (c) und (VK1) entbehrlich.

Der zweite Flüssigkeitsweg beginnt mit der Pumpen/Substratkammer (PK) führt über die Dosierkammer (DK) und die Kapillare (Kap) zur Überlaufkammer (ÜK) und von dort direkt zur Ventilkammer (VK2). Der gemeinsame Teil des Weges beider Flüssigkeiten führt von der Ventilkammer (VK2) zur Kammer (d), in welcher sich beispielsweise ein Festphasen-gebundener Reaktionspartner befindet, von dort zur Auffangkammer (AK) und zur Küvette (K). Druckausgleichbohrungen B1 bis B4 verhindern die Bildung eines den Flüssigkeitstransport störenden Luftpolsters.

Dieses Einsatzelement ist geeignet, Flüssigkeit ausschließlich durch Variation der Zentrifugalkraft gesteuert zu transportieren, während in den Kapillaren (Auslauf von VK1, VK2; sowie Kap) jeweils eine konstante Kapillarkraft vorliegt.

Das erfindungsgemäße Verfahren läßt sich auf diesem Einsatzelement wie folgt durchführen:

Durch ein geeignetes Zentrifugierprogramm mit abwechselnder Erhöhung und Erniedrigung der Drehzahl wird einerseits die in Kammer (a) aufgebrachte Probe über (b), (VK1), (c) und (VK2) in die Kammer (d) gebracht, welche als Trennsäule wirkt und in welcher an die saugfähige Füllung ein Partner einer Immunreaktion unlöslich fixiert ist. Beispielsweise besteht die saugfähige Füllung aus einem Cellulosevlies, an welches der Immunreaktionspartner kovalent fixiert ist. Durch das Zentrifugierprogramm wird andererseits die in die Pumpenkammer (PK) eingegebene Flüssigkeit in der Dosierkammer in mehrere einzelne, kleinere und gleiche Volumina separiert, deren erste in der Überlaufkammer (ÜK) gesammelt werden. Bei Fortgang des Zentrifugierprogrammes wird die Kammer (d) ausgeschleudert, die Überlaufkammer (ÜK) wird durch weitere Substratlösungsvolumina zum Überlaufen gebracht und über die Ventilkammer (VK2) werden dann einzelne Portionen Substratlösung in die Kammer (d) gebracht, die vor dem Ankommen der nächsten Flüssigkeitsportion jeweils auszentrifugiert wird. Nach mehreren derartigen Waschvorgängen verbleibt eine Portion der Substratflüssigkeit für eine bestimmte Reaktionszeit in der Kammer (d) und reagiert dort mit trägergebundenem Markierungsenzym unter Farbentwicklung. Die Restmenge der Probe, sowie die einzelnen, aus (d) ausgeschleuderten Waschvolumina füllen die als Überlaufkammer ausgebildete Auffangkammer (AK) fast vollständig, so daß die am Schluß auf (d) reagierende Substratflüssigkeits-Portion größtenteils an AK vorbei in die Küvette (K) strömt und dort in bekannter Weise gemessen wird. Das Volumen der Auffangkammer AK ist abgestellt auf die Volumina von

Probelösung, Dosierkammer und Überlaufkammer unter Berücksichtigung des Volumens der Küvette (K) und der gewünschten Anzahl Wasch-Schlucke, abhängig vom Volumen der Substratflüssigkeit.

Im folgenden werden die Funktionen im einzelnen beschrieben:

Dosierkammer DK und Kapillare Kap

Die Pumpen/Substratkammer PK enthält Vliesmaterial, das in der Lage ist, die gesamte Substratmenge in sich aufzusaugen. Das Vlies bildet eine Vielzahl zusammenhängender kleiner Hohlräume, welche dem Flüssigkeitstransport einen Widerstand entgegensetzen. Bei Zentrifugation mit hoher Drehzahl wird Flüssigkeit aus dem Pumpenkammervlies in die Dosierkammer DK gedrückt und füllt diese vollständig. Die unter der Substratkammer befindliche Kapillare Kap wird unter dieser wirkenden Zentrifugalkraft als kommunizierendes Gefäß zur Substratkammer nur zu einem Teil in der oberen Hälfte gefüllt.

Beim Absinken der Drehzahl bis zum Stand wird die Flüssigkeit in der Pumpenkammer PK von dem Vlies wieder aufgesaugt. Es kommt am Eingang zur Dosierkammer DK zu einem Flüssigkeitsabriß, erst dann kommt es infolge der Schwerkraft der Flüssigkeit in der Dosierkammer DK und der Saugkraft der Kapillare Kap zu einer vollständigen Füllung von Kap. Bei anschließendem Zentrifugieren mit niedriger Drehzahl wirkt die Kapillare als Heber und saugt die Dosierkammer DK leer.

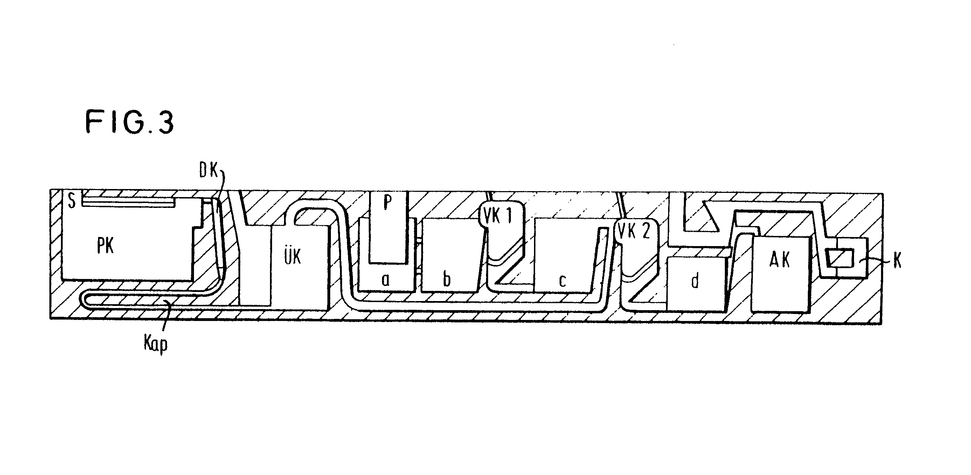

Eine andere Ausgestaltung der Überlaufkammer (ÜK) (Fig. 3) gestattet das Leersaugen von DK und Kap im Stillstand durch Kontakt der Vliese in ÜK mit dem Auslaufende von Kap.

Erst bei erneuter hoher Drehzahl läßt das Vlies in der Pumpenkammer PK die Flüssigkeit wieder los und es kommt zu einer neuen Füllung der Dosierkammer DK und teilweisen Füllung der Kapillare Kap. Dieser Vorgang wird mehrmals wiederholt bis zur gewünschten Portionierung des gesamten Flüssigkeitsvolumens der Pumpenkammer PK.

Überlaufkammern ÜK und AK

Wie schon erwähnt, funktioniert auch die Auffangkammer AK als Überlaufkammer. Der Eingang in diese Überlaufkammern ist so gestaltet, daß die Flüssigkeit unter der vorherrschenden Zentrifugalkraft in diese eingeleitet wird und die darin befindliche Luft vollständig verdrängt werden kann.

Sobald die Kammer völlig gefüllt ist, strömt die weitere Flüssigkeit an ihr vorbei.

Durch die Überlaufkammer ÜK ist es möglich, trotz gleichzeitiger Füllung des Einsatzelements mit Proben- und Substratflüssigkeit in die beiden getrennten Kammern PK und P in einem geeigneten Zentrifugierprogramm die Probe über die beiden Ventilkammern VK1 und VK2 auf das Feld d zu transportieren, während die Substratflüssigkeit in der Überlaufkammer ÜK zunächst noch zurückgehalten wird.

Eine alternative Ausgestaltung von ÜK ist in Fig. 3 gezeigt. Hier wird ebenfalls ein definiertes Volumen Flüssigkeit zurückgehalten, jedoch wird die Kammer von allen dosierten Schlucken durchströmt. Der Vorteil dieser Kammer liegt im Zurückhalten der durchlaufenden Flüssigkeit bei niedrigen Drehzahlen.

Ferner wird durch die Auffangkammer AK die Möglichkeit eröffnet, den Rest der Probenflüssigkeit und die einzelnen Waschvolumina aufzufangen und erst die auf der Trennsäule d reagierte Substratlösung in die Küvette K zu leiten.

Fig. 2 zeigt ein weiteres Einsatzelement mit zwei Flüssigkeitswegen, bei welchen der zweite Flüssigkeitsweg vollständig einen Teil des längeren ersten Flüssigkeitsweges darstellt. Bei dieser Ausführungsform befindet sich in der Kammer (c) an die feste Phase, z. B. in Form eines Cellulosevlieses, unlöslich gebunden Hapten oder Antigen. Die Kammer (c) weist eine Öffnung auf, durch welche eine Flüssigkeit einpipettiert werden kann. Der zweite Flüssigkeitsweg besteht daher aus der Kammer (c), die mit einer Auffangkammer (AK) in Verbindung steht. Der erste Flüssigkeitsweg beginnt mit der Probekammer (P), geht von P dort weiter zur Kammer (a), in der sich auf einem Vlies ein Trockenreagenz befindet, von dort zur Ventilkammer (VK1), in der die Reaktion zwischen Probe und aus Kammer (a) herausgelösten Reagenz ablaufen kann. Von Ventilkammer (VK1) geht der Flüssigkeitsweg weiter zur Kammer (b), welche z. B. enzymmarkierte Antikörper bzw. Fragmente derselben enthalten kann. Sie werden ebenfalls von der Probeflüssigkeit herausgelöst und reagieren in der anschließenden Ventilkammer (VK2). Danach gelangen sie in die Kammer (c) und damit in den gemeinsamen Teil beider Flüssigkeitswege. Es führt der Weiterweg zur Kammer (d), in der sich ein Trockensubstrat befindet, anschließend folgt die Ventilkammer (VK3) sowie eine von dort zur Meßküvette (K) führende Verbindungsstrecke.

Im Betrieb läuft das Verfahren mit dieser Ausführungsform des Einsatzelementes wie folgt ab:
In die Matrixkammer (c) einpipettierte Waschflüssigkeit wird während der ersten Zentrifugation in die Auffangkammer (AK) geschleudert. Hierdurch werden von der Matrix Haptenmoleküle, deren Bindung zur Matrix sich während der Lagerung gelöst hatte, entfernt.

Gleichzeitig wird Probeflüssigkeit in die Probekammer (P) eingegeben. Während der ersten Zentrifugation überströmt die Probeflüssigkeit die Kammer (a), löst dabei die dort befindlichen Reagenzien und gelangt in die Ventilkammer (VK1), wo die Umsetzung stattfindet. Bei gleichzeitigen Reaktionen sind Feld a und VK1 entbehrlich, es genügt, die Aufbringung des gesamten Reagenz für die Vorreaktion auf Feld b und Inkubation in VK2.

Bei Herabsetzung der Zentrifugalkraft überwiegt die Kapillarkraft und die Probelösung wird aus VK1 abgesaugt und gelangt in die Kammer (b). Dort werden enzymmarkierte Antikörper oder bevorzugt Antikörperfragmente abgelöst. Bei Erhöhung der Zentrifugalkraft gelangt die Flüssigkeit in die Ventilkammer (VK2) und kann dort reagieren solange die Zentrifugalkraft aufrechterhalten wird. Bei Herabsetzung der Zentrifugalkraft (Stillstand) wird die Probe abgesaugt durch die Kapillarkraft und gelangt in die Kammer (c), also in den gemeinsamen Weg beider Flüssigkeiten. Dort kann die Reaktion ablaufen, bis in einem weiteren Schritt durch Erhöhung der Zentrifugalkraft die Flüssigkeit aus (c) ausgeschleudert wird und teilweise in (AK) bis zu deren Füllung gelangt und teilweise direkt weitergeleitet wird zur Kammer (d). Dort wird immer noch unter dem Einfluß dere Zentrifugalkraft das Enzymnachweisreagenz herausgelöst und gleich in die Ventilkammer VK3 weitertransportiert, wo die Farbbildungsreaktion beginnt oder abläuft. Bei Herabsetzung der Zentrifugalkraft wird durch die Kapillarkraft die Lösung aus VK3 abgesaugt und bei erneuter Erhöhung der Zentrifugalkraft in die Küvette (K) transportiert und ausgemessen.

Weitere Gegenstände der Erfindung sind die in den Fig. 1 bis 3 dargestellten, für die Durchführung des erfindungsgemäßen Verfahrens geeigneten bevorzugten Ausführungsformen von Rotoreinsatzelementen für Zentrifugalanalysatoren.

Ein erfindungsgemäßes Rotoreinsatzelement für Zentrifugalanalysenautomaten zur Durchführung des Verfahrens, enthaltend einen Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial weiter außen führt, wenn das Einsatzelement auf dem Rotor befestigt ist, ist gekennzeichnet durch wenigstens eine weitere Kammer die von außen mit einer von der Probenflüssigkeit verschiedenen Flüssigkeit versehen werden kann und einen Transportweg, der von dieser Kammer zur Meßkammer führt und mit dem Probenflüssigkeitstransportweg mindestens teilweise identisch ist.

Eine bevorzugte Ausführungsform eines derartigen Einsatzelementes ist dadurch gekennzeichnet, daß der Probenflüssigkeitstransportweg von der Probenauftragskammer (P) über ein oder mehrere mit saugfähigem Material gefüllte Trockenreagenz enthaltende Kammern (a), gegebenenfalls (b), (c) oder weitere und eine zwischen den Kammern, z. B. (b) und (c) angeordnete erste Ventilkammer (VK1) zu einer zweiten Ventilkammer (VK2) und von dieser über eine mit trägerfixierten Immunreaktanten versehene, als Trennsäule ausgebildete Kammer (d) und über eine Auffangkammer (AK) zur Meßkammer (K) führt und zur Aufnahme einer weiteren Flüssigkeit eine Pumpen/Substratkammer (PK) vorgesehen ist, welche über eine aus Dosierkammer (DK) und Kapillare (Kap) bestehende Dosiereinrichtung und eine Überlaufkammer (ÜK) mit der zweiten Ventilkammer (VK2) verbunden ist.

Eine alternative Ausführungsform des erfindungsgemäßen Einsatzelements ist dadurch gekennzeichnet, daß der Probenflüssigkeitstransportweg von der Probenauftragskammer (P) über die mit saugfähigem Material gefüllten Trockenreagenzien enthaltenden Kammern (a) und (b) sowie Kammer (c), die als Trennsäule mit trägerfixiertem Immunreaktanten ausgebildet ist, zwischen denen die Ventilkammern (VK1 bzw. VK2) angeordnet sind, vorbei an Auffangkammer (AK) zur Trockenreagenz enthaltenden, mit saugfähigem Material gefüllten Kammer (d) und weiter über eine dritte Ventilkammer (VK3) zur Meßkammer (K) führt und die Kammer (c) eine Öffnung nach außen aufweist, durch welche eine weitere Flüssigkeit zugegeben werden kann, welche über (c) in die Auffangkammer (AK) gelangt.

Vorzugsweise weisen die Ventilkammern und die Meßkammer des erfindungsgemäßen Einsatzelements Entlüftungskanäle auf.

Die Funktion der erfindungsgemäßen Einsatzelemente im Rahmen der Durchführung des erfindungsgemäßen Verfahrens wird durch die folgenden Beispiele näher erläutert.

**Beispiel 1**

Bestimmung von TSH als Antigen unter Verwendung eines Einsatzelementes gemäß Fig. 1.

Bestückung der Kammern des Rotoreinsatzelementes.

Substratlösung:
0,9 NaCl
Na-HEPES, 70 mM, pH 7,25
Borsäure, 5 mM
Mg(OH)$_2$, 0,5 mM
RSA (Rinderserumalbumin) 0,3 %
Chlorphenolrotgalactosid,50 mM (Substrat)
PK:
0,2 % Tween 20
Vliese zur Gesamtdicke von 3,5 mm
ÜK:
Vliese zur Gesamtdicke von 2 mm
a:
2 Vliese je 0,7 mm dick
Na-HEPES, 50 mm, pH 7,25 (37° C)
Tween 20, 0,1 %
Lactose, 3 %
c:
1 Vlies, 1 mm dick
Anti-TSH-monoklonaler Antikörper Fab-Fragment, gekuppelt an β-Galactosidase 200 mU (Fab-E) monoklonaler Antikörper gegen TSH 250 ng
HEPES, 200 mM, pH 7,25
Mg-Aspartat, 20 mM
Saccharose, 6 %
RSA, 1 %
Tween 20, 0,1 %
d:
2 Vliese, 0,7 mm dick
beladen mit 5 mg Schaf-IgG-anti-Maus-Fc$_\gamma$ - (Antikörper gegen Anti-TSH )
gewaschen mit:
Na-PO$_4$, 10 mM, pH 6,5
NaCl, 154 mM
RSA 1 %

AK:
4 Vliese zur Gesamtdicke von 3,5 mm.

Flüssigkeitspipettierungen

In die Pumpen/Substratkammer werden 286 µl Substratlösung pipettiert. 40 µl Probe werden durch eine Öffnung am oberen Rand direkt auf das Feld a pipettiert. Die Probe ist in diesem Falle unverdünnt.

Reaktionsführung

Durch ein geeignetes Reaktionsprogramm, wo hohe Drehzahlen mit Stillstand abwechseln, werden jetzt Probe und Substrat in Richtung Trennmatrix und Küvette gefördert. Im folgenden steht Zentrifugation für hohe Drehzahl, zwischengeschaltete Schritte mit niedriger Drehzahl dienen der sensibleren Steuerung des Flüssigkeitstransportes, ändern aber nichts an der prinzipiellen Funktion. Die Substrat/Waschlösung wird durch die Dosierkapillare (DK) in gleich große Schlucke (Portionen) geteilt. Verwendet wird ein Disposable nach Fig. 1.
1. Zentrifugation
Probe und Probepuffer werden in VK1 geschleudert, der erste Schluck ist in der Dosierkammer DK.
1. Stillstand
Probe läuft auf Feld c und löst Anti-TSH und Fab-E (Konjugat). Der 1. Schluck Substratlösung geht in die Überlaufkammer ÜK.
2. Zentrifugation
TSH, Anti-TSH und Fab-E gelangen in die VK 2, es wird 5 min zentrifugiert, um die homogene Vorreaktion ablaufen zu lassen, in der sich der Komplex Anti-TSH.TSH.Fab-E bildet. Der 1. Schluck Substratlösung wird in ÜK zurückgehalten, der 2. Schluck Substratlösung geht in die Dosierkammer DK.
2. Stillstand
Probeseitig wird die Flüssigkeit auf Feld d transportiert, d. h. die in der vorigen Reaktion gebildeten Komplexe erreichen jetzt die Matrix; es folgt ein Stillstand von 5 Minuten, während der Zeit die Komplexe an die Matrix gebunden werden. An die Matrix fest gebunden sind Antikörper gegen Anti-TSH. Es bindet auch nicht komplexiertes Anti-TSH. Am Ende der Reaktion befindet sich noch nicht komplexiertes Fab-E ungebunden in der Lösung auf der Matrix. Der 2. Schluck Substratlösung geht in die ÜK.
3. Zentrifugation
Die aus dem Probekanal gekommene Flüssigkeit wird in die Auffangkammer (AK) zentrifugiert, mit ihr der Überschuß an Fab-E.
Der 2. Substratschluck wird in ÜK gehalten.
Der 3. Substratschluck befindet sich in Dosierkammer DK.
3. Stillstand
Der 3. Substratschluck wird nach ÜK befördert.
4. Zentrifugation
Schluck 4 nach DK
Schluck 3 nach VK 2
4. Stillstand
Schluck 4 nach ÜK
Schluck 3 nach Feld d

Der 1. Waschschluck befindet sich auf der Matrix.

5. Zentrifugation
Schluck 5 nach DK
Schluck 4 nach VK2
Schluck 3 nach AK
5. Stillstand
Schluck 5 nach ÜK
Schluck 4 nach d
2. Waschschluck auf der Matrix
6. Zentrifugation
Schluck 6 nach DK
Schluck 5 nach VK2
Schluck 4 nach AK
6. Stillstand
Schluck 6 nach ÜK
Schluck 5 nach d
3. Waschschluck auf der Matrix
7. Zentrifugation
Schluck 7 nach DK
Schluck 6 nach VK2
Schluck 5 nach AK
7. Stillstand
Schluck 7 nach ÜK
Schluck 6 nach d
4. Waschschluck auf der Matrix
8. Zentrifugation
Schluck 8 nach DK
Schluck 7 nach VK2
Schluck 6 nach AK
8. Stillstand
Schluck 8 nach ÜK
Schluck 7 nach d
Nachweisschluck auf der Matrix:
In 5 Minuten Reaktion wird das Substrat von dem auf der Matrix gebundenen Enzym, d. h. einer Enzymmenge, die durch die Komplexbildung proportional zur eingesetzten Menge TSH ist, gespalten und es bildet sich die zu messende Farbe.
9. Zentrifugation
Die von der Matrix kommende Flüssigkeit füllt mit einem ersten Aliquot die AK vollständig und der Rest wird in die Küvette gefördert. In der Küvette erfolgt die Messung der gebildeten Farbe bei 578 nm.

Der beschriebene Reaktionsablauf eignet sich für alle polyvalenten Antigene. Ausgetauscht werden müssen lediglich die auf Vlies c befindlichen Antikörper gegen das zu bestimmende Antigen, also Anti-Ag und Fab-E. Es werden 3 Phasen von je 5 Minuten verwendet, und zwar:
homogene Mischung in VK 2
Matrixbindung der entstandenen Komplexe auf d,
Farbentwicklung durch das auf der Matrix gebundene Enzym auf d.
Zwischen Matrixreaktion und Farbentwicklung liegen mehrere Waschschritte, um überschüssiges Enzym zu entfernen.

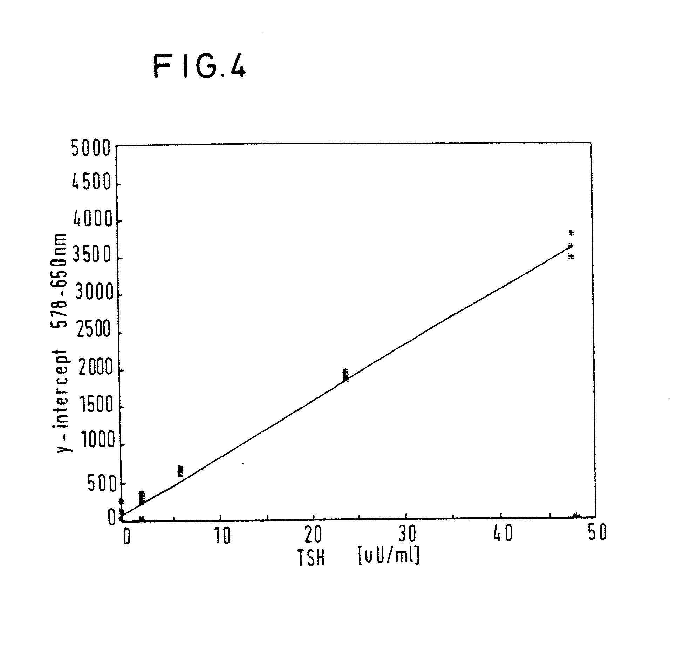

Durch die Proportionalität der auf der Matrix gebundenen Enzymmenge zur Konzentration des Analyten werden lineare Eichkurven erhalten. Eine Eichkurve für TSH ist in der Fig. 4 der Zeichnung dargestellt.

Wird als Markierungsenzym nicht $\beta$-Galactosidase verwendet, so ist in der Substratkammer Chlorphenolrot-Galactosid durch ein entsprechendes, für das andere Enzym geeignetes Substrat zu ersetzen. Diese Substrate sind dem Fachmann geläufig.

**Beispiel 2**

Bestimmung von HCG

Das Verfahren ist analog Beispiel 1, jedoch wird auf Vlies c als Konjugat eine Fab-Fragment-Enzym Verbindung eines monoklonalen Antikörpers (MAK) gegen HCG verwendet. Ferner kommt ein zweiter MAK gegen HCG zum Einsatz.

**Beispiel 3**

Bestimmung von AFP

Das Verfahren ist, wie im Beispiel 1, die verwendeten Antikörper auf Vlies c sind gegen AFP gerichtet. Wegen der höheren Konzentration von AFP im Serum wird die Probe im Verhältnis 1:10 mit physiologischer Kochsalzlösung vorverdünnt.

**Beispiel 4**

Bestimmung von T3 als Beispiel eines Haptens, also eines monovalenten Antigens unter Verwendung eines Einsatzelements gemäß Fig. 2

Bestückung des Einsatzelementes

a:    2 Vliese Puffer, 1 Vlies Ablösereagenz, je 0,5 mm dick
      Na-HEPES, 125 mM, pH 7,25
      Tween 20, 0,25 %
      ANS 0,06 % (ANS = Anilino Naphthalin Sulfonsäure),
      Tween 20, 0,01 %
b:    2 Leervliese, 1 Konjugatvlies, jeweils 0,5 mm dick
      Anti-T3-monoklonaler Antikörper Fab-Fragment, gebunden an $\beta$-Galactosidase 1,6 mU (Fab-E)
      Na-HEPES, 100 mm, pH 7,25
      Polyoxygelatine 1 %
      Mg-Aspartat, 5 mm
c:    Trenn-Matrix, 2 Vliese, je 0,7 mm dick

T3, unlöslich gebunden an das Matrixvlies

AK: 1 Vlies von 1 mm Dicke

d: 1 Vlies von 0,5 mm Dicke

Na-HEPES, 100 mM, pH 7,25

Borsäure, 5 mM

Chlorphenolrotgalactosid 18 mM

## Flüssigkeitspipettierungen

Es werden 5 $\mu$l Probelösung in die Probenauftragskammer P pipettiert, gefolgt von 50 $\mu$l Diluens (physiologische Kochsalzlösung). Die Mischung der Komponenten erfolgt durch den Pipettiervorgang. Auf Feld c werden 40 $\mu$l Diluens pipettiert.

## Reaktionsführung

Das Zentrifugationsprogramm ist identisch mit dem Programm gemäß Beispiel 1 bis einschließlich dem 3. Stillstand. Danach erfolgt direkt die Messung der Reaktion bei Meßdrehzahl.

1. Zentrifugation

Die auf die Matrix (c) pipettierte Flüssigkeit wird in die Auffangkammer (AK) geschleudert. Durch diesen Waschvorgang der Matrix werden Haptenmoleküle, deren Bindung zur Matrix sich während der Lagerung gelöst hatte, entfernt. Diese Moleküle würden ansonsten wie Probe wirken und das Ergebnis verfälschen. Die Probeflüssigkeit überströmt gleichzeitig Feld a, löst dabei die dort befindlichen Reagenzien und es kann in der Ventilkammer VK1 eine Vorreaktion stattfinden. Die Ablösereaktion, bei der ANS das T3 aus der Bindung mit den Bindungsproteinen (vorwiegend TBG) löst, muß nicht vollständig sein, da eine Weiterreaktion in VK2 hier möglich ist.

1. Stillstand

Die Probe geht auf Feld b, hier wird das Fab-Enzym-Konjugat abgelöst.

2. Zentrifugation

Die Probe wird in VK 2 befördert. Diese Zentrifugation wird für 5 Minuten aufrechterhalten. Dabei reagiert das T3 aus der Probe mit dem Fab-Enzym-Konjugat (Fab-E) zu dem Komplex T3·Fab-E.

2. Stillstand

Die Probe geht auf Vlies c. Hier bindet überschüssiges Fab-E an die Matrix über das matrixgebundene T3. Diese Reaktion dauert 5 Minuten.

3. Zentrifugation

Der erste Teil der Flüssigkeit füllt die Auffangkammer vollständig, der größere Teil wird über Vlies d in die VK 3 geschleudert. Von d wird dabei das Substrat heruntergelöst.

3. Stillstand

Die reagierende Lösung verläßt VK 3.

Meßzentrifugation

Die Lösung wird in die Küvette transportiert und die Reaktion absorptionsphotometrisch bei 578 nm verfolgt. Die Konjugatmoleküle, welche T3 aus der Probe gebunden hatten, konnten die Matrix passieren und es befindet sich jetzt eine der Konzentration T3 entsprechende Menge Enzym in der Küvette. Die gemessene Farbzunahme pro Zeiteinheit ist daher ein Maß für die Konzentration T3 in der Probe.

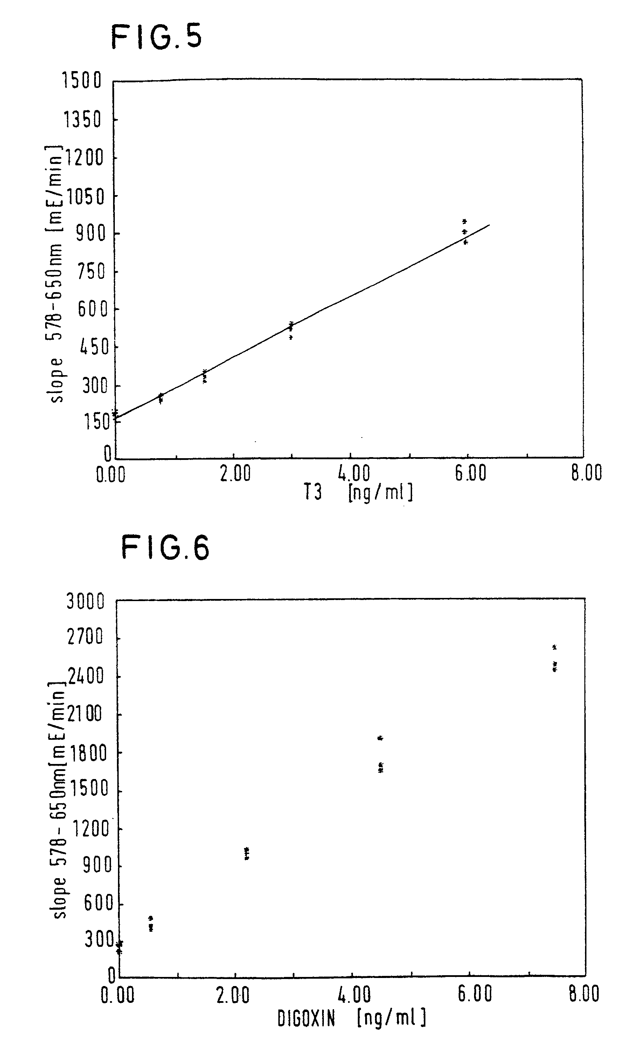

Wegen der Proportionalität zwischen Enzym und Analyt werden lineare Eichkurven erhalten. Dies zeigt Fig. 5 der Zeichnung.

## Beispiel 5

Bestimmung von Digoxin

Das Verfahren ist analog dem Beispiel 4, jedoch werden für das Konjugat Fab-Fragmente aus einem Antikörper gegen Digoxin verwendet. Die Matrix besteht in diesem Fall aus an die Festphase gebundenem Digoxin. Eine so erhaltene Eichkurve zeigt Fig. 6.

## Patentansprüche

1. Verfahren zur Durchführung analytischer Bestimmungen durch Mischen und Inkubieren einer Probelösung mit wenigstens einem Reagenz und Messung eines Parameters im Reaktionsgemisch, wobei man die Probelösung zuerst zu einem löslichen Trockenreagenz transportiert unter wenigstens teilweiser Auflösung des letzteren und dann unter dem Einfluß eines Kraftänderungszyklus weitertransportiert, bei welchem man eine Zentrifugalkraft und eine weitere Kraft derart auf die Lösung einwirken läßt, daß abwechselnd eine Kraft die andere Kraft überwiegt und den Weitertransport und die Transportrichtung bestimmt, **dadurch gekennzeichnet**, daß man wenigstens eine weitere Flüssigkeit auf einem Teil des Weges getrennt von der Probelösung und/oder weiteren Flüssigkeiten gleichzeitig transportiert und die Transportwege derart auf die Kraftänderungszyklen abstellt, daß die verschiedenen Flüssigkeiten den gemeinsamen Teil des Transportweges zeitlich getrennt passieren.

2. Verfahren nach Anspruch 1, **dadurch gekennzeichnet**, daß wenigstens einer der getrennten Transportwege so gestaltet wird, daß die darin transportierte Flüssigkeit bei jedem Kraftänderungszyklus nur einen Teil des vom Weg der anderen Flüssigkeit getrennten Weges zurücklegt.

3. Verfahren nach Anspruch 2, **dadurch gekennzeichnet,** daß in beiden Transportwegen je Kraftänderungszyklus nur ein Teilweg von der Flüssigkeit zurückgelegt wird und einer der getrennten Flüssigkeitstransportwege mehr Kraftänderungszyklen bis zum Erreichen des für mindestens zwei der Flüssigkeiten gemeinsamen Transportweges benötigt als der andere Transportweg.

4. Verfahren nach Anspruch 2 oder 3, **dadurch gekennzeichnet,** daß man im gemeinsamen Transportweg wenigstens ein festes reaktives unlösliches Material vorsieht, an welchem man wenigstens zwei Reaktionen zeitlich voneinander getrennt ablaufen läßt, indem man es zuerst mit der Lösung kontaktiert, die weniger Kraftänderungszyklen bis zum Erreichen dieses Trägermaterials benötigt und danach mit der weiteren Flüssigkeit kontaktiert.

5. Verfahren nach Anspruch 4, **dadurch gekennzeichnet,** daß man im Transportweg der Probelösung als lösliches Trockenreagenz ein markiertes immunologisch aktives Material verwendet, welches mit der zu bestimmenden Substanz bindefähig ist, im gemeinsamen Transportweg als festes reaktives Material ein damit immunologisch bindefähiges Material und im getrennten Transportweg für die weitere Flüssigkeit ein System zur Bestimmung des Markierungssystems als festes lösliches Trockenreagenz anordnet.

6. Rotoreinsatzelement für Zentrifugalanalysenautomaten zur Durchführung des Verfahrens gemäß einem der vorhergehenden Ansprüche, enthaltend einen Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial weiter außen führt, wenn das Einsatzelement auf dem Rotor befestigt ist, **gekennzeichnet durch** wenigstens eine weitere Kammer die von außen mit einer von der Probenflüssigkeit verschiedenen Flüssigkeit versehen werden kann und einen Transportweg, der von dieser Kammer zur Meßkammer führt und mit dem Probenflüssigkeitstransportweg mindestens teilweise identisch ist.

7. Einsatzelement nach Anspruch 6, **dadurch gekennzeichnet,** daß der Probenflüssigkeitstransportweg von der Probenauftragskammer (P) über mindestens eine mit saugfähigem Material gefüllte Trockenreagenz enthaltende Kammer (a), gegebenenfalls (b) und/oder (c) und gegebenenfalls eine zwischen den Kammern angeordnete erste Ventilkammer (VK1) zu einer zweiten Ventilkammer (VK2) und von dieser über eine mit trägerfixiertem Immunreaktanten versehene, als Trennsäule ausgebildete Kammer (d) und über eine Auffangkammer (AK) zur Meßkammer (K) führt und zur Aufnahme einer weiteren Flüssigkeit eine als Pumpenkammer ausgebildete Substratkammer (PK) vorgesehen ist, welche über eine aus Dosierkammer (DK) und Kapillare (Kap) bestehende Dosiereinrichtung und eine Überlaufkammer (ÜK) mit der zweiten Ventilkammer (VK2) verbunden ist.

8. Einsatzelement nach Anspruch 6, **dadurch gekennzeichnet,** daß der Probenflüssigkeitstransportweg von der Probenauftragskammer (P) über die mit saugfähigem Material gefüllten Trockenreagenzien enthaltenden Kammern (a), (b) und (c), zwischen denen die Ventilkammern (VK1 bzw. VK2) angeordnet sind und wobei Kammer (c) als Trennsäule mit trägerfixiertem Immunreaktanten ausgebildet ist, über eine Auffangkammer (AK) zur Trockenreagenz enthaltenden, mit saugfähigem Material gefüllten Kammer (d) und weiter über eine dritte Ventilkammer (VK3) zur Meßkammer (K) führt und die Kammer (c) eine Öffnung nach außen aufweist, durch welche eine weitere Flüssigkeit zugegeben werden kann.

9. Einsatzelement nach einem der Ansprüche 6 bis 8, **dadurch gekennzeichnet,** daß die Ventilkammern und die Meßkammer Entlüftungskanäle aufweisen.

**Claims**

1. Process for the carrying out of analytical determinations by mixing and incubation of a sample solution with at least one reagent and measurement of a parameter in the reaction mixture, whereby one first transports the sample solution to a soluble dry reagent with at least partial dissolving of the latter and then further transports under the influence of a force-change cycle in the case of which one allows a centrifugal force and a further force to act upon the solution in such a manner that, alternatingly, one force predominates the other force and determines the further transport and the transport direction, characterised in that one simultaneously transports at least one further liquid on a part of the path separate from the sample solution and/or further liquids and adapts the transport paths to the force-change cycles in such a manner that the different liquids pass the common part of the transport path chronologically separated.

2. Process according to claim 1, characterised in that at least one of the separate transport paths is so constructed that the liquid transported therein passes only a part of the path separate from the path of the other liquid in the case of each force-change cycle.

3. Process according to claim 2, characterised in that, in both transport paths, for each force-change cycle only a partial path of the liquid is passed and one of the separate liquid transport paths requires more force-change cycles up to the reaching of the transport path common for at least two of the liquids than does the other transport path.

4. Process according to claim 2 or 3, characterised in that, in the common transport path, one provides at least two reactions to take place chronologically separated from one another in that one first contacts it with the solution which requires fewer force-change cycles up to the reaching of this carrier material and thereafter contacts with the further liquid.

5. Process according to claim 4, characterised in that, in the transport path of the sample solution, one uses, as soluble dry reagent, a labelled, immunologically-active material which is bindable with the substance to be determined, in the common transport path as solid reactive material a material immunologically bindable therewith and in the separate transport path for the further liquid arranges a system for the determination of the labelling system as solid, soluble dry reagent.

6. Rotor insert element for centrifugal automatic analysers for the carrying out of the process according to one of the preceding claims, containing a formed body which has a sample application chamber which is connected with a plurality of reagent zones, each of which contains an absorbent material impregnated with a particular reagent, at least one mixing valve chamber and a measurement chamber which together form a sample liquid transport path which runs from radially inwardly to radially further outside when the insert element is fixed on to the rotor, characterised by at least one further chamber, which can be provided from outside with a liquid different from the sample liquid, and a transport path which leads from this chamber to the measurement chamber and is at least partly identical with the sample liquid transport path.

7. Insert element according to claim 6, characterised in that the sample transport path leads from the sample application chamber (P) via at least one chamber (a) filled with absorbent material containing dry reagent, possibly (b) and/or (c) and possibly a first valve chamber (VK1) arranged between the chambers to a second valve chamber (VK2) and from this via a chamber (d), constructed as a separating column, provided with carrier-fixed immune reactants and via a reception chamber (AK) to the measurement chamber (K) and, for the reception of a further liquid, there is provided a substrate chamber (PK) constructed as a pump chamber which is connected with the second valve chamber (VK2) via a dosing device consisting of dosaging chamber (DK) and an overflow chamber (UK).

8. Insert element according to claim 6, characterised in that the sample liquid transport path leads from the sample application chamber (P) via the chambers (a), (b) and (c) filled with absorbent material containing dry reagents, between which are arranged the valve chambers (VK1 and VK2, respectively) and whereby the chamber (c) is constructed as separating column with carrier-fixed immune reactants, via a reception chamber (AK) to the chamber (d) filled with absorbent material containing dry reagent and further via a third valve chamber (VK3) to the measurement chamber (K) and the chamber (c) has an opening to the outside through which a further liquid can be introduced.

9. Insert element according to one of claims 6 to 8, characterised in that the valve chambers and the measurement chamber have aeration canals.

**Revendications**

1. Procédé pour l'exécution de déterminations analytiques par mélange et incubation d'une solution échantillon avec au moins un réactif et mesure d'un paramètre dans le mélange réactionnel, en transportant tout d'abord la solution échantillon jusqu'à un réactif sec soluble avec dissolution au moins partielle de ce dernier puis en continuant à la transporter sous l'influence d'un cycle de modification de forces, dans lequel on fait agir sur la solution une force centrifuge et une autre force de telle manière que tour à tour une force domine l'autre et détermine la suite du transport et la direction du transport, caractérisé en ce que l'on transporte au moins un autre liquide sur une partie de la voie séparément de la solution échantillon et/ou l'on transporte simultanément d'autres liquides et l'on adapte les voies de transport en fonction des cycles de modification de forces de telle manière que les différents liquides parcourent la partie commune de la voie de transport séparément dans le temps.

2. Procédé selon la revendication 1, caractérisé en ce qu'au moins l'une des voies de transport séparées est agencée de telle manière que le liquide qui y est transporté ne parcourt à chaque cycle de modification de forces qu'une partie de la voie séparée de la voie de l'autre liquide.

3. Procédé selon la revendication 2, caractérisé en ce que dans les deux voies de transport seul une voie partielle est parcourue par le liquide à chaque cycle de modification de forces et l'une des voies de transport de liquide séparées nécessite un plus grand nombre de cycles de modification de forces que l'autre voie de transport pour atteindre la voie de transport commune pour au moins deux des liquides.

4. Procédé selon la revendication 2 ou 3, caractérisé en ce que l'on prévoit dans la voie de transport commune au moins un matériau réactif solide insoluble au niveau duquel on fait se dérouler au moins deux réactions séparées l'une de l'autre dans le temps, en le mettant tout d'abord en contact avec la solution qui nécessite un moins grand nombre de cycles de modification de forces pour atteindre ce matériau support puis avec l'autre liquide.

5. Procédé selon la revendication 4, caractérisé en ce que l'on utilise comme réactif sec soluble dans la voie de transport de la solution échantillon un matériau immunologiquement actif marqué qui est capable de se lier à la substance à déterminer, comme matériau réactif solide dans la voie de transport commune un matériau capable de liaison immunologique avec celle-ci et dans la voie de transport séparée pour l'autre liquide un système pour la détermination du système de marquage en tant que réactif sec solide soluble.

6. Elément d'insertion de rotor pour dispositifs automatiques d'analyse centrifuge pour la mise en oeuvre du procédé selon l'une des revendications précédentes, contenant une pièce moulée qui présente une chambre d'application de solution qui est reliée à une multiplicité de champs de réactif qui contiennent chacun un matériau support absorbant imprégné d'un réactif déterminé, au moins une chambre de vanne mélangeuse et une chambre de mesure qui forment ensemble une voie de transport de liquide échantillon qui conduit radialement de l'intérieur vers l'extérieur, lorsque l'élément d'insertion est fixé sur le rotor, caractérisé par au moins une autre chambre qui peut être munie de l'extérieur d'un liquide différent du liquide échantillon et par une voie de transport qui conduit de cette chambre à la chambre de mesure et qui est au moins partiellement identique à la voie de transport de liquide échantillon.

7. Elément d'insertion selon la revendication 6, caractérisé en ce que la voie de transport de liquide échantillon conduit de la chambre d'application d'échantillon (P), par l'intermédiaire d'au moins une chambre (a), éventuellement (b) et/ou (c), contenant un réactif sec et remplie de matériau absorbant, et éventuellement d'une première chambre de vanne (VK1) disposée entre les chambres, à une deuxième chambre de vanne (VK2) et de celle-ci, par l'intermédiaire d'une chambre (d) sous forme d'une colonne de séparation munie d'un corps réagissant immunologique fixé sur support, et par l'intermédiaire d'une chambre collectrice (AK), à la chambre de mesure (K) et pour la réception d'un autre liquide une chambre de substrat (PK) sous forme de chambre de pompe, qui est reliée par l'intermédiaire d'un dispositif de dosage consistant en une chambre de dosage (DK) et de capillaires (Kap), et d'une chambre de trop-plein (UK) à la seconde chambre de vanne (VK2).

8. Elément d'insertion selon la revendication 6, caractérisé en ce que la voie de transport de liquide échantillon conduit de la chambre d'application d'échantillon (P), par l'intermédiaire des chambres (a), (b) et (c) remplies de matériau absorbant et contenant des réactifs secs, entre lesquelles sont disposées les chambres de vanne (VK1 et VK2) et la chambre (c) étant sous forme d'une colonne de séparation avec des corps réagissants immunologiques fixés sur support, par l'intermédiaire d'une chambre collectrice (AK), à la chambre (d) remplie de matériau absorbant et contenant un réactif sec et ensuite à la chambre de mesure (K) par l'intermédiaire d'une troisième chambre de vanne (VK3), et la chambre (c) présente une ouverture vers l'extérieur par laquelle il est possible d'introduire un autre liquide.

9. Elément d'insertion selon l'une des revendications 6 à 8, caractérisé en ce que les chambres de vanne et la chambre de mesure présentent des canaux d'évacuation d'air.

FIG.1

FIG.2

FIG.3

# FIG.4

# FIG.5

# FIG.6